# EUROPÄISCHE PATENTSCHRIFT

(11) **EP 1 302 172 B1**
(45) Veröffentlichungstag und Bekanntmachung des Hinweises auf die Patenterteilung: **05.03.2008**
(21) Anmeldenummer: 01123369.9
(22) Anmeldetag: 10.10.2001
(51) Int. Cl.: A61B 19/00

(54) **Medizinisches Instrument mit berührungsempfindlicher Spitze**
Medical instrument having a touch sensitive tip
Instrument médical avec une pointe sensible au toucher

(43) Veröffentlichungstag der Anmeldung: 16.04.2003
(73) Patentinhaber: BrainLAB AG, 85551 Kirchheim/Heimstetten (DE)
(72) Erfinder: Bertram, Michael, 85570 Markt Schwaben (DE); Birkenbach, Rainer, 85445 Aufkirchen (DE); Vilsmeier, Stefan, 6330 Kufstein (AT)
(74) Vertreter: Schwabe - Sandmair - Marx

(56) Entgegenhaltungen:
- EP-A- 0 000 556
- WO-A-00/78209
- WO-A-99/38449
- US-A- 4 399 823
- US-A- 5 339 799
- US-A- 5 718 228
- US-A- 6 006 126
- US-A- 2001 025 183

## Beschreibung

Die Erfindung betrifft ein medizinisches Instrument mit einem Instrumentenkörper, einer Instrumentenspitze und einer Trackingeinrichtung, die es gestattet, die räumliche Position des Instrumentes mittels einer Navigationseinrichtung zu bestimmen. Solche medizinischen Instrumente werden derzeit in einer Ausbildung als Zeigegerät oder "Pointer" beispielsweise verwendet, um bestimmte Punkte eines Patienten oder auf dem Patienten angebrachte Marker mit der Spitze anzufahren, um diese Punkte bzw. Marker mittels eines Navigationssystems räumlich zu registrieren. Dabei kennt das Navigationssystem, weil es über die Trackingeinrichtung am Instrument die Position des Instruments bzw. des Pointers selbst kennt, nachdem Berühren zum Beispiel eines Markers mit der Spitze des Instruments auch die Position des Markers, und durch das Anfahren mehrerer Marker können die aktuelle Position des Patienten und diejenige des zu behandelnden Körperteils registriert werden. Ein solches Registrierungssystem ist beispielsweise aus der DE 19 639 615 C2 bekannt. Nachteilig ist dabei einerseits, dass dem Navigationssystem immer durch eine separate Aktion mitgeteilt werden muss, dass gerade ein Marker zur Registrierung angefahren worden ist, beispielsweise durch eine Eingabe am Navigationssystem. Andererseits verschiebt sich der Marker möglicherweise bei der Berührung durch die Pointerspitze, so dass Ungenauigkeiten entstehen können.

Ein weiteres bekanntes Referenzierungssystem verwendet keine Pointer mehr, sonder strahlt Lichtpunkte auf die Hautoberfläche des Patienten auf, dessen Außenkontur vorher mit einer Bilderfassungstechnik, zum Beispiel CT oder MR, aufgezeichnet wurde. Die Reflexionen mehrerer Lichtpunkte auf der Hautoberfläche des Patienten werden durch ein Kamerasystem dreidimensional erfasst, und hieraus wird eine Kontur errechnet, die dann über sogenanntes "surface-matching" der entsprechenden Kontur des Patienten aus der Bilderfassung zugeordnet wird. Auch dadurch lässt sich die momentane Lage des Patienten im Navigationssystem ermitteln. Der Nachteil dieser Technik besteht darin, dass sie nur mit Navigationssystemen verwendet werden kann, die optische Positionserfassungen aufweisen, zum Beispiel mit Kameras. Es werden jedoch in jüngerer Zeit immer häufiger Navigations- bzw. Trackingsysteme verwendet, die auf einer magnetischen Erfassung basieren, also Instrumente im Bereich eines aufgebauten Magnetfeldes verfolgen, wobei in den Instrumenten Spulen angeordnet sind, deren Position im Magnetfeld erfasst werden kann. Bei solchen Systemen kann die oben angesprochene berührungslose Technik mit der Aufstrahlung von Lichtpunkten nicht angewendet werden, da kein Kamerasystem vorhanden ist, welches die Lichtreflexionen erfassen kann. Es muss deshalb wieder auf ein System zurückgegriffen werden, bei dem mehrere Punkte auf der Oberfläche des Patienten mittels einer Pointerspitze angefahren werden, um dann aus der dadurch entstehenden Teilkontur über das surface-matching eine Konturenzuordnung durchzuführen. Gerade wenn aber Punkte auf der Hautoberfläche mit Pointerspitzen angefahren werden, kommt es zu relativ großen Ungenauigkeiten, da die Hautoberfläche sehr nachgiebig ist und oftmals "eingedellte" Punkte erfasst werden, wenn dem Navigationssystem nach dem Anfahren eines Punktes auf der Hautoberfläche mitgeteilt wird, dass gerade in diesem Moment eine Punktregistrierung erfolgt (beispielsweise über Knopfdruck am Instrument oder an einer Eingabetaste des Navigationssystems). Die herkömmlichen Techniken führen hierbei also einerseits zu Ungenauigkeiten und sind andererseits bedienungsaufwendig.

Die US 2001/0025183 A1 beschreibt die Ausrüstung einer Ultraschallsonde mit einem Berührungssensor an der Spitze, zur Verfolgung eines beweglichen Ziels

Es ist die Aufgabe der vorliegenden Erfindung, ein medizinisches Instrument bereitzustellen, welches die oben genannten Nachteile des Standes der Technik überwindet. Insbesondere soll die Berührung der Instrumentenspitze mit einem Objekt genau und unaufwändig erfasst werden können.

Diese Aufgabe wird erfindungsgemäß durch ein medizinisches Instrument gemäß dem Anspruch 1 gelöst. Mit anderen Worten hat das Instrument eine "taktile" Spitze, mittels der automatisch erfasst wird, ob diese Spitze ein Objekt berührt.

Ein Vorteil eines erfindungsgemäßen Instrumentes liegt darin, dass sofort bei der Berührung der Spitze mit einem Objekt bzw. Gegenstand, beispielsweise der Hautoberfläche des Patienten, eine solche Berührung erfasst werden kann, also noch bevor das Objekt selbst durch die Berührung verschoben wird. Dies erhöht die Genauigkeit der Erfassung wesentlich. Weiterhin vorteilhaft ist die Tatsache, dass kein separater Schutz von Seiten des Instrumentenbedieners notwendig ist, um eine solche Berührung einem angeschlossenen System mitzuteilen; dies kann durch den Berührungssensor selbst erledigt werden, wodurch eine sehr einfache Bedienbarkeit entsteht.

Ein weiterer Vorteil besteht noch darin, dass ein Sensor gewählt werden kann, der nur bei bestimmten Objektoberflächen ein Signal erzeugt, so dass Fehlsignale ausgeschlossen werden können.

Das erfindungsgemäße medizinische Instrument umfasst eine Übertragungseinrichtung, die ein Signal überträgt, wenn die Berührung erfasst wird. Hierbei wird der Berührungssensor als Berührungsschalter, der bei Berührung ein Signal ausgibt, betrieben. Dieses Signal kann auf verschiedene Weise zu einem angeschlossenen System übertragen werden, nämlich beispielsweise durch ein am Instrument angebrachtes Kabel oder über einen Sender für die drahtlose Signalübertragung.

Um zu vermeiden, dass der Berührungssensor ständig und kurz hintereinander Signale abgibt, ist es von Vorteil, der Übertragungseinrichtung bzw. dem Sensor eine Steuerung zuzuordnen, die eine wiederholte Signalübertragung erst nach einer vorbestimmten, verstrichenen Zeitspanne zulässt. Wichtig ist eine solche Ausführung dann, wenn beispielweise ein Zeigegerät bzw. ein Pointer mit einer erfindungsgemäßen taktilen Spitze ausgestattet wird. Wenn dieser Pointer im Rahmen einer Magnettracking-Registrierung auf einen Punkt an der Hautoberfläche des Patienten geführt wird, wird sofort bei Berührung der Spitze mit der Hautoberfläche ein Signal abgegeben, und zwar noch bevor die Haut mit der Instrumentenspitze eingedellt werden kann. Man erhält deshalb eine sehr genaue Punktregistrierung. Mit der oben angesprochenen Steuerung der Signalübertragung, die eine wiederholte Übertragung erst nach einer vorbestimmten, verstrichenen Zeitspanne zulässt, lässt es sich nun verhindern, dass während des Eindrückens der Hautoberfläche immer weiter Registrierungssignale abgegeben werden, welche die Registrierung insgesamt verfälschen könnten.

Von besonderem Vorteil ist eine Ausführungsform des erfindungsgemäßen Instruments bei der die Spitze vom Instrumentenkörper abnehmbar ist. Eine solche abnehmbare Spitze kann nämlich einzeln sterilisiert oder steril gehalten werden. Gemäß einer weiteren Ausführungsform ist ferner möglich, solche Spitzen gleich als Wegwerfartikel auszubilden, die beispielsweise steril verpackt werden könnten und nach dem Gebrauch nicht mehr sterilisiert werden müssten.

Die Trackingeinrichtung, d. h. diejenige Vorrichtung am Instrument, die dessen Verfolgung im Rahmen eines Navigationssystems gestattet, kann eine Magnet-Trackingeinrichtung sein, wobei das Instrument dabei Magnettrackingspulen umfasst. Eine solche Ausführungsform ist dann vorteilhaft, wenn kein optisches Trackingsystem zur Verfügung steht. Jedoch kann die vorliegende Erfindung auch bei Instrumenten mit optischen Trackingeinrichtungen, also beispielsweise aktiven oder passiven Markierungsanordnungen, Anwendung finden; auch eine Kombination ist denkbar.

Der Berührungssensor kann ein elektrischer Widerstandssensor, ein kapazitiver Sensor oder ein mechanischer Sensor sein, bzw. eine Kombination aus diesen Sensorarten, wie sie dem Anwendungsfall am besten entspricht. In bevorzugter Ausführung ist das medizinische Instrument als Registrierungspointer für Instrumententrackingsystemen ausgeführt. Die oben schon angesprochene einfache Bedienbarkeit wirkt sich in diesem Rahmen bei optischen und magnetbasierten Trackingsystemen aus, da separat zu bedienende Schalter am Instrument, Fußschalter oder Eingabeschalter am Navigationssystem nicht mehr betätigt werden müssen. Ein erfindungsgemäß ausgestatteter Registrierungspointer hat insbesondere den Vorteil, dass er eine surface-matching-Registrierung ohne Markierungen auch für Magnet-Trackingsysteme ermöglicht, und zwar mit hoher Genauigkeit.

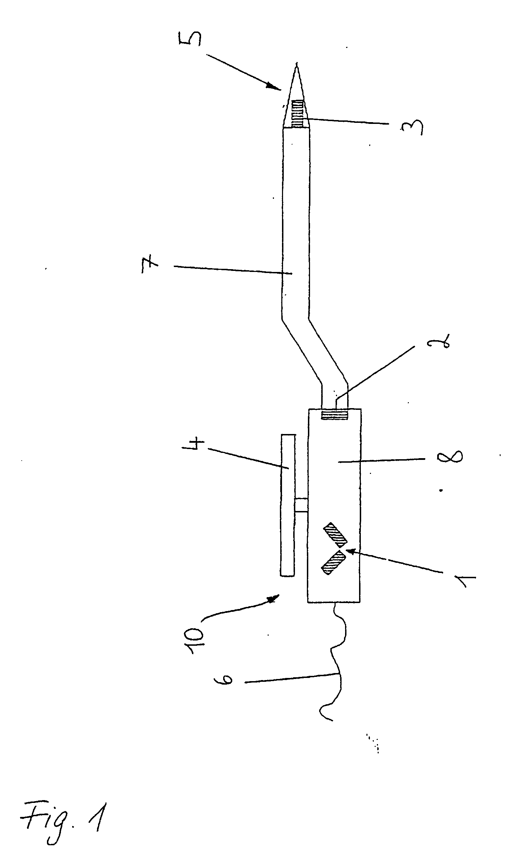

Die Erfindung wird im weiteren anhand der beiliegenden Zeichnung näher erläutert, die schematisch ein medizinisches Instrument in einer Ausführung als Registrierungspointer zeigt.

Der Pointer in der Figur ist insgesamt mit dem Bezugszeichen 10 bezeichnet. Er weißt einen Grundkörper 8, eine vordere Verlängerung 7 und eine Spitze 5 auf. Der dargestellte Pointer 10 kann als Magnettracking-Pointer verwendet werden, und hierzu kann er mit kleinen Induktionsspulen versehen werden, wie dies beispielsweise an in mehreren Stellen aufgezeichnet ist, nämlich beim Spulenpaar 1, im Instrumentenkörper 8, bei der Spule 2 am Übergang zwischen Instrumentenkörper 8 und Verlängerung 7 sowie direkt in der Spitze 5, wo die Spule 3 dargestellt ist. Das Spulenpaar 1 bildet einen sogenannten "sechsdimensionalen" Sensor für Magnettracking, die Spulen 2 und 3 bilden je einen "fünfdimensionalen", zusammen einen sogenannten "sechsdimensionalen" Magnettrackingsensor. Das Spulenpaar 2, 3 genügt alleine, ebenso wie das Spulenpaar 1, als Sensoreinheit. Ebenfalls in der Zeichnung ist noch zusätzlich eine optische Trackingvorrichtung mit dem Bezugszeichen 4 dargestellt, die alternativ oder in Kombination mit der Magnet-Trackingeinrichtung verwendet werden kann. Die optische Trackingeinrichtung 4 kann beispielsweise aus einer aktiven oder passiven Markeranordnung bestehen, d. h. aus Markern, die selbst Lichtsignale abstrahlen oder aus Reflexionsmarkern.

In der Zeichnung ist mit dem Bezugszeichen 6 ein Kabel angedeutet, über das ein Signal zu einem Navigationssystem übertragen werden kann, wenn die taktile Spitze 5 einen Berührungsvorgang meldet. Nicht dargestellt, jedoch im Rahmen der vorliegenden Erfindung vorstellbar, ist es anstelle des Kabels einen Sender zu verwenden, der das Berührungssignal drahtlos überträgt.

Die Spitze 5 ist eine taktile Spitze, d. h. sie weist einen berührungsempfindlichen Sensor auf, der irgendein in der Technik bekannter, berührungsempfindlicher Sensor sein kann. Je nach Anwendungsfall kann er zum Beispiel ein elektrischer Widerstandssensor, ein kapazitiver Sensor oder ein mechanischer Sensor sein. Sie arbeitet wie ein kleiner, sensibler Schalter oder elektronischer Sensor, der dazu in der Lage ist, einen Oberflächenkontakt zu erfassen. Der Sensor kann den Schalter beinhalten; es können aber auch Schalter und Sensor getrennt vorliegen. Auch der Schalter kann zum Beispiel mechanisch oder elektrisch bzw. magnetisch funktionieren. Jedes Mal, wenn der Pointer die Oberfläche des zu registrierenden Objektes, also beispielsweise einen Punkt auf der Hautoberfläche berührt, wird ein Signal erzeugt bzw. gesetzt, das digital oder elektrisch sein kann. Bei einem kapazitiven Sensor fließt dabei zum Beispiel ein Wechselstrom über den Patienten, der durch Kapazitäten von etwa 1 pF begrenzt wird. Der Sensor setzt dann einen Signalaufgang von "High" auf "Low", oder umgekehrt.

Somit kann der Berührungssensor an der Oberfläche für eine hochgenaue Konturerfassung verwendet werden, da eine Punktregistrierung immer schon stattgefunden hat, bevor die Hautoberfläche eingedrückt werden könnte. Aus der räumlichen Position des Pointers 10, die durch die Trackingeinrichtung (Spulen- und/oder Markeranordnung) erhalten wird, kann eine Oberfläche des Objektes und ihre Raumposition bestimmt werden. Kein weiterer manueller Schalter ist notwendig. Wenn eine ausreichende Anzahl von Oberflächenpunkten durch Berührung registriert worden sind, gestattet ein surface-matching mit den vorher aus einer Bilderfassung erhaltenen Patienteninformationen eine markerlose Registrierung.

## Patentansprüche

1. Medizinisches Instrument mit einem Instrumentenkörper (8), einer Instrumentenspitze (5) und einer Trackingeinrichtung (1, 2, 4), wobei die Instrumentenspitze (5) mit einem Berührungssensor (3) ausgestattet ist, der eine Berührung der Spitze (5) mit einem Objekt erfasst, wobei das Instrument ferner eine Übertragungseinrichtung (6) umfasst, die ein Signal überträgt, wenn die Berührung erfasst wird, **dadurch gekennzeichnet, dass** das Instrument ein Zeigegerät bzw. Pointer (10) ist, dessen räumliche Position bei der Berührung mittels der Trackingeinrichtung erhalten wird.

2. Instrument nach Anspruch 1, **dadurch gekennzeichnet, dass** die Übertragungseinrichtung ein Kabel (6) ist.

3. Instrument nach Anspruch 1, **dadurch gekennzeichnet, dass** die Übertragungseinrichtung ein Sender zur drahtlosen Signalübertragung ist.

4. Instrument nach einem der Ansprüche 1 bis 3, **dadurch gekennzeichnet, dass** der Übertragungseinrichtung bzw. dem Sensor (3) eine Steuerung zugeordnet ist, die eine wiederholte Signalübertragung erst nach einer vorbestimmten, verstrichenen Zeitspanne zulässt.

5. Instrument nach einem der Ansprüche 1 bis 4, **dadurch gekennzeichnet, dass** die Spitze (5) vom Instrumentenkörper (8) abnehmbar ist.

6. Instrument nach Anspruch 5, **dadurch gekennzeichnet, dass** die Spitze (5) als Wegwerfartikel ausgebildet ist.

7. Instrument nach einem der Ansprüche 1 bis 6, **dadurch gekennzeichnet, dass** die Trackingeinrichtung Magnettrackingspulen (1, 2, 3) umfasst.

8. Instrument nach einem der Ansprüche 1 bis 7, **dadurch gekennzeichnet, dass** die Trackingeinrichtung eine optisch erfassbare Markeranordnung umfasst.

9. Instrument nach einem der Ansprüche 1 bis 8, **dadurch gekennzeichnet, dass** der Sensor ein elektrischer Widerstandssensor, ein kapazitiver Sensor oder ein mechanischer Sensor ist, bzw. eine Kombination aus diesen Sensorarten.

10. Instrument nach einem der Ansprüche 1 bis 9, **dadurch gekennzeichnet, dass** es als Registrierungspointer für Instrumententrackingsysteme ausgeführt ist.

## Claims

1. A medical instrument comprising an instrument body (8), an instrument tip (5) and a tracking means (1, 2, 4), wherein the instrument tip (5) is fitted with a touch-sensor (3) which detects a contact between the tip (5) and an object, wherein the instrument further comprises a transmission means (6) which transmits a signal when contact is detected, **characterised in that** the instrument is an indicator device or pointer (10), the spatial position of which is obtained by means of the tracking means during contact.

2. The instrument according to claim 1, **characterised in that** the transmission means is a cable (6).

3. The instrument according to claim 1, **characterised in that** the transmission means is a sender for transmitting signals wirelessly.

4. The instrument according to any one of claims 1 to 3, **characterised in that** a control system is assigned to the transmission means or sensor (3), which only allows it to transmit another signal after a predetermined period of time has elapsed.

5. The instrument according to any one of claims 1 to 4, **characterised in that** the tip (5) may be removed from the instrument body (8).

6. The instrument according to claim 5, **characterised in that** the tip (5) is realised as a disposable article.

7. The instrument according to any one of claims 1 to 6, **characterised in that** the tracking means comprises magnetic tracking coils (1, 2, 3).

8. The instrument according to any one of claims 1 to 7, **characterised in that** the tracking means comprises an optically detectable arrangement of markers.

9. The instrument according to any one of claims 1 to 8, **characterised in that** the sensor is an electrical resistance sensor, a capacity sensor or a mechanical sensor, or a combination of these types of sensors.

10. The instrument according to any one of claims 1 to 9, **characterised in that** it is realised as a registering pointer for instrument tracking systems.

## Revendications

1. Instrument médical avec un corps d'instrument (8), une pointe d'instrument (5) et un dispositif de poursuite (1, 2, 4), dans lequel la pointe d'instrument (5) est équipée d'un capteur de contact (3) qui détecte un contact de la pointe (5) avec un objet, dans lequel l'instrument comprend en outre un dispositif de transmission (6) qui transmet un signal lorsque le contact est détecté, **caractérisé en ce que** l'instrument est un appareil d'affichage ou un index (10) dont la position spatiale est obtenue lors du contact au moyen du dispositif de poursuite.

2. Instrument selon la revendication 1, **caractérisé en ce que** le dispositif de poursuite est un câble (6).

3. Instrument selon la revendication 1, **caractérisé en ce que** le dispositif de transmission est un émetteur pour la transmission sans fil de signaux.

4. Instrument selon l'une des revendications 1 à 3, **caractérisé en ce qu'**au dispositif de transmission ou au capteur (3) est associée une commande qui permet une nouvelle transmission de signaux seulement après un intervalle de temps prédéterminé, écoulé.

5. Instrument selon l'une des revendications 1 à 4, **caractérisé en ce que** la pointe (5) du corps d'instrument (8) est amovible.

6. Instrument selon la revendication 5, **caractérisé en ce que** la pointe (5) est réalisée sous la forme d'un article jetable.

7. Instrument selon l'une des revendications 1 à 6, **caractérisé en ce que** le dispositif de poursuite comprend des bobines de poursuite magnétiques (1, 2, 3).

8. Instrument selon l'une des revendications 1 à 7, **caractérisé en ce que** le dispositif de poursuite comprend un agencement de repères pouvant être détecté optiquement.

9. Instrument selon l'une des revendications 1 à 8, **caractérisé en ce que** le capteur est un capteur de résistance électrique, un capteur capacitif ou un capteur mécanique, ou une combinaison de ces types de capteur.

10. Instrument selon l'une des revendications 1 à 9, **caractérisé en ce qu'**il est réalisé sous la forme d'un index d'enregistrement pour systèmes de poursuite d'instrument.
